# EUROPEAN PATENT SPECIFICATION

(11) **EP 0 517 975 B1**
(45) Date of publication and mention of the grant of the patent: **28.08.1996**
(21) Application number: 91305354.2
(22) Date of filing: 13.06.1991
(51) Int. Cl.: A61B 17/072

(54) **Surgical stapler**
Chirurgisches Klammernahtgerät
Instrument d'agrafage chirurgicale

(43) Date of publication of application: 16.12.1992
(73) Proprietor: Takase, Haruo, Tokyo (JP)
(72) Inventor: Takase, Haruo, Tokyo (JP)
(74) Representative: Wood, Anthony Charles

(56) References cited:
- WO-A-83/00614
- DE-C- 406 832
- FR-A- 2 452 275
- US-A- 4 241 861

## Description

This invention relates to a surgical stapler for suturing tissues in a surgical operation with square U-shaped staples, and more particularly to a surgical suturing stapler capable of being readily loaded with staples by use of a detachable staple cartridge.

The inventor of this invention has formerly proposed surgical suturing staplers for inserting square U-shaped staples into a bodily part to stitch a wound or the like and arrest hemorrhage. (Japanese Patent Public Discl. No. SHO 63(1988)-270040(A) and Japanese U.M. Pub. Discl. No. HEI 1(1990)-62810(A))

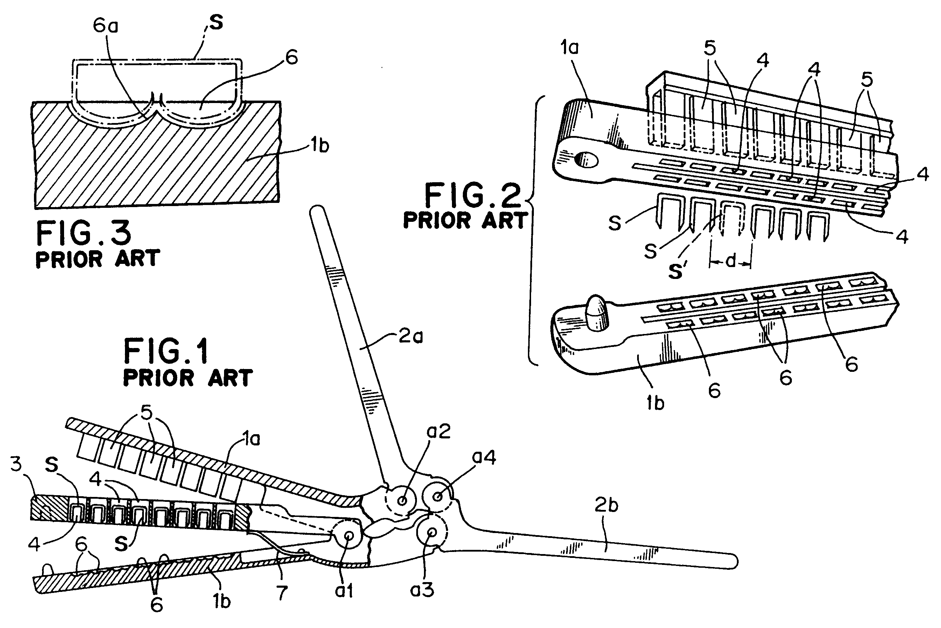

The prior art surgical suturing staplers including the staplers proposed by the inventor as noted above can discharge a number of U-shaped staples in one lot into a bodily part as illustrated in Figures 1 and 2. The stapler of this type comprises a pair of jaws 1a, 1b pivotally supported by an axial pin a1, a pair of handles 2a, 2b having tip portions rotatably connected to the jaws 1a, 1b through axial pins a2, a3 and pivotally supported by an axial pin a4, and a staple magazine 3. As shown in Figure 2, two parallel rows of staple chambers 4 in which the staples (S) are loaded are formed in the staple magazine 3.

The upper jaw 1a has extruding pieces 5 to be plunged into the staple chambers 4 in the staple magazine 3 to push the staples (S) out of the staple chambers 4 toward the lower jaw 1b. The lower jaw 1b is provided in its upper surface with anvil grooves 6 opposite to the staple chambers 4 in the staple magazine so as to bend inwardly the legs of each staple (S) being thrust thereinto.

A bodily part such as the edges of a wound in a surgical operation can be sutured with the staples (S) merely by gripping the handles 2a, 2b with putting the bodily part between the upper and lower jaws 1a and 1b. When gripping the handles 2a, 2b, the upper and lower jaws 1a, 1b are rotated around the axial pin a1 and closed to force the staples out of the staple chambers 4. The staples thus forced out are pressed against the lower jaw 1b to cause the legs of the staples to be thrust into the anvil grooves 6 and bent inwardly. Thus, the tissues are kept in a state pressed with the staples so as to arrest hemorrhage.

The surgical stapler noted above provides advantageous suturing of the tissues of a living body, as even a large incision part can be easily stitched with a number of staples in one operation. This stapler has been found a big help in a surgical operation requiring prompt treatment, but is disadvantageous in that the work of being loaded with the staples (S) turns out to be a very troublesome chore in a surgical operation. That is, the staples (S) must be inserted one by one into the staple chambers 4 formed in the staple magazine 3 by hand in such a state that the upper and lower jaws 1a, 1b are opened as wide as possible. This work requires much time and labor and proves to be troublesome, so that it is substantially impossible to load the staple magazine with the staples while being operated.

Furthermore, the conventional surgical stapler entails problem such as difficulty in visually discerning whether the staples are infallibly placed in all the staple chambers 4 of the staple magazine. There is a possibility that the staple falls out of the staple chamber in the magazine accidentally, suffering a disadvantage that a wound or other possible tissues would be incompletely sutured owing to the falling-off of the staple. For instance, when the staple (S') as shown in Figure 2 fails of being charged in or falls out of the staple chamber, the part (d) indicated in the drawing cannot provide effects of suturing tissues and arresting haemorrhage.

In the surgical stapler of this type, the staple is retained inside the staple chamber 4 by frictional force produced by the inner surrounding surface of the staple chamber. However, the friction force by which the staple is retained would gradually decrease during the course of prolonged service, and therefore, the staple retained would easily fall out of the staple chamber with slight shock or other possible external force.

The anvil groove 6 formed in the lower jaw 1b of the conventional surgical stapler has a bottom surface 6a shaped in a substantial ω as shown in Figure 3. The staple being thrust into the anvil groove is bent inwardly along the bottom surface 6a of the anvil groove 6 with the pointed ends of the legs being curved upwardly as indicated by imaginary lines in the drawing. Namely, the staple is bent in a general B-shape. Therefore, the grasping force at the central portion of the bent staple becomes weaker, so that the effect of arresting haemorrhage at that portion would be reduced. Thus, the conventional stapler entailed a disadvantage that the staples cannot be readily loaded nor reliably retained within the staple chambers in the staple magazine, and the legs of the staples cannot be bent suitably, to thereby suture tissues incompletely.

FR-A-2,452,275 discloses a surgical stapler having the features of the preamble of Claim 1.

In view of the drawbacks of the conventional surgical stapler as mentioned above, it is an object of the present invention to provide a surgical suturing stapler capable of being readily and promptly loaded with staples even in the middle of a surgical operation and securely retaining the staples.

Another object of the present invention is to provide a surgical stapler having a staple cartridge capable of reliably retaining staples therein and visually discerning the retained staples so as to suture tissues infallibly.

A further object of the present invention is to provide a surgical stapler capable of suitably bending the legs of the staples inserted into tissues to bring about an effect of steadily arresting haemorrhage.

The invention provides a surgical stapler according to Claim 1.

Upon permitting the staple cartridge to be loaded with the staples and fitted into cartridge holding member, the staples are discharged out of the cartridge by stapler extruding pieces which are thrust into the staple chambers in the staple cartridge when the first and second jaws are closed. The legs of the staples thus discharged pierce through tissues or the like interposed between the staple cartridge and the second jaw and are forced into the anvil grooves to thereby be bent inwardly.

Since the staple cartridge is detachably fitted into the cartridge holding member, the staples can easily be inserted in the staple cartridge and visually discerned.

By using the magnetic staple cartridge which is magnetized, the staples loaded into the staple cartridge are magnetically retained to be prevented from falling out of the staple cartridge accidentally.

The other objects and features of the present invention will now be explained in detail with reference to the accompanying drawings, wherein:

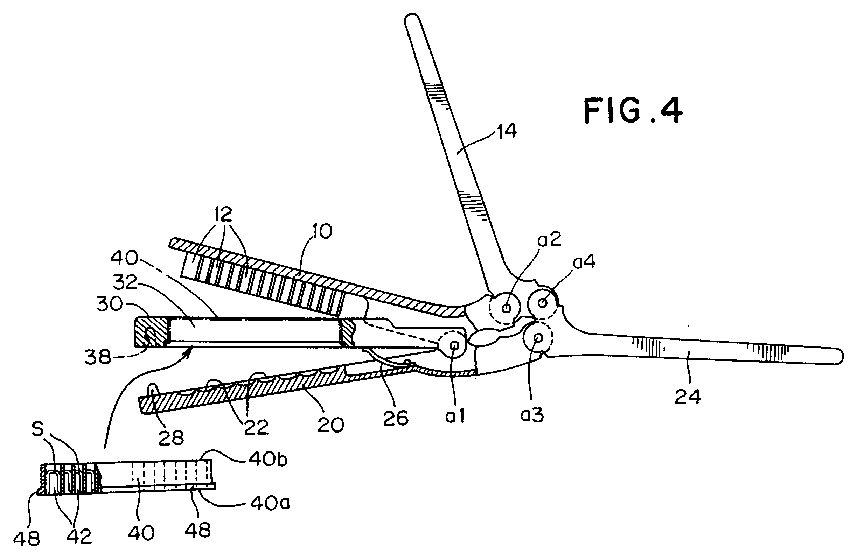

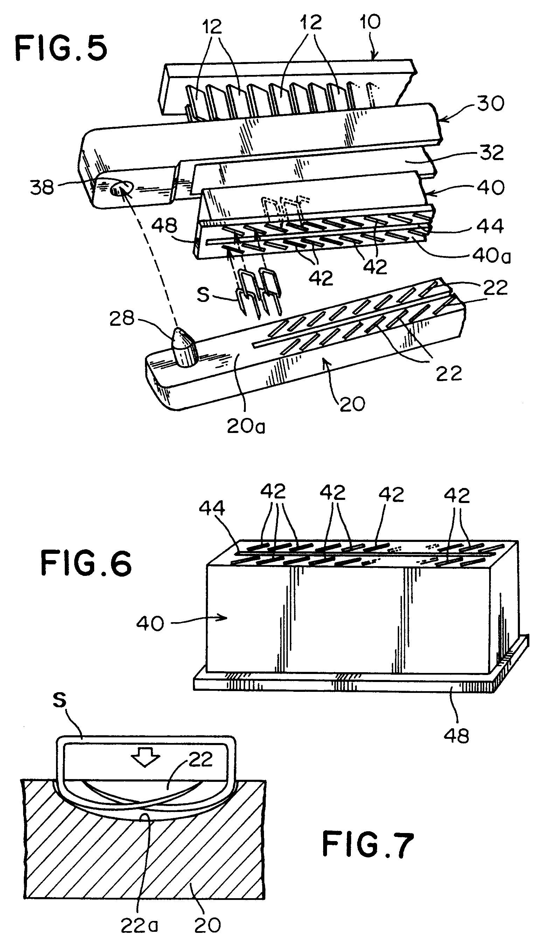

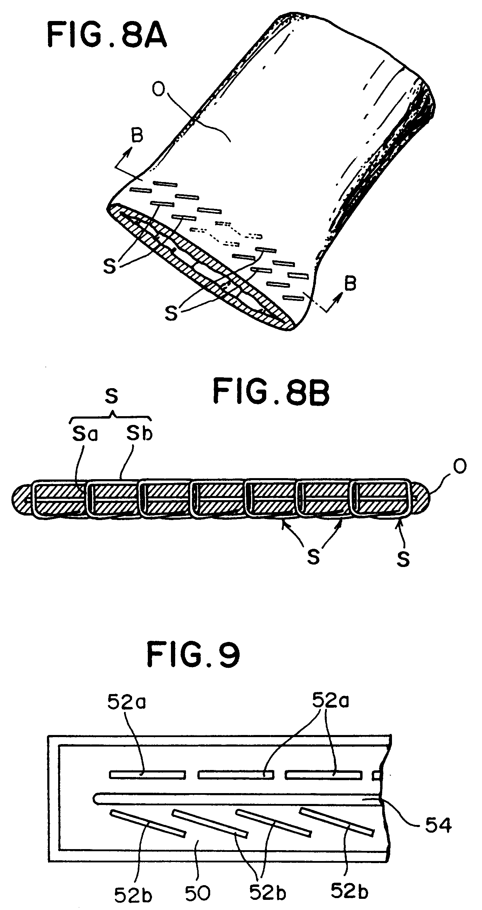

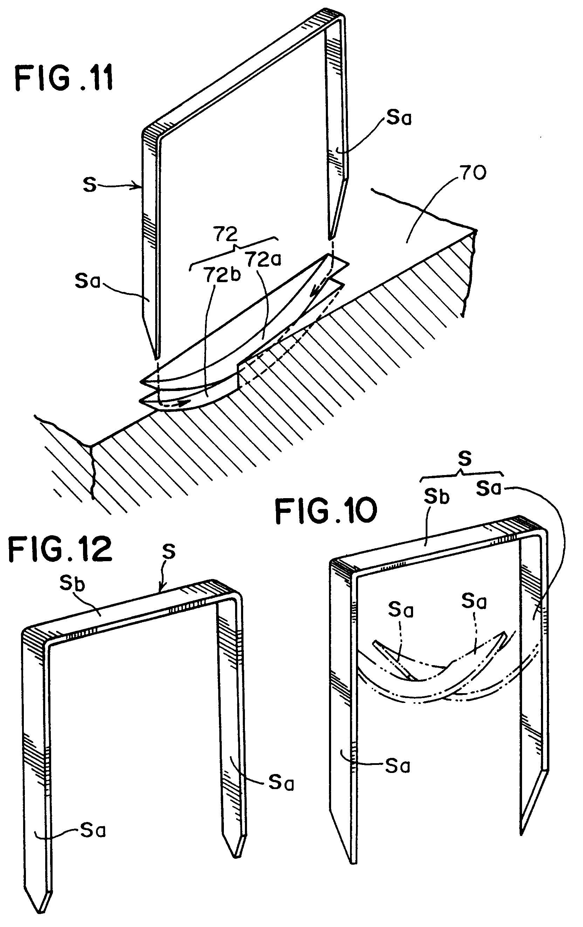

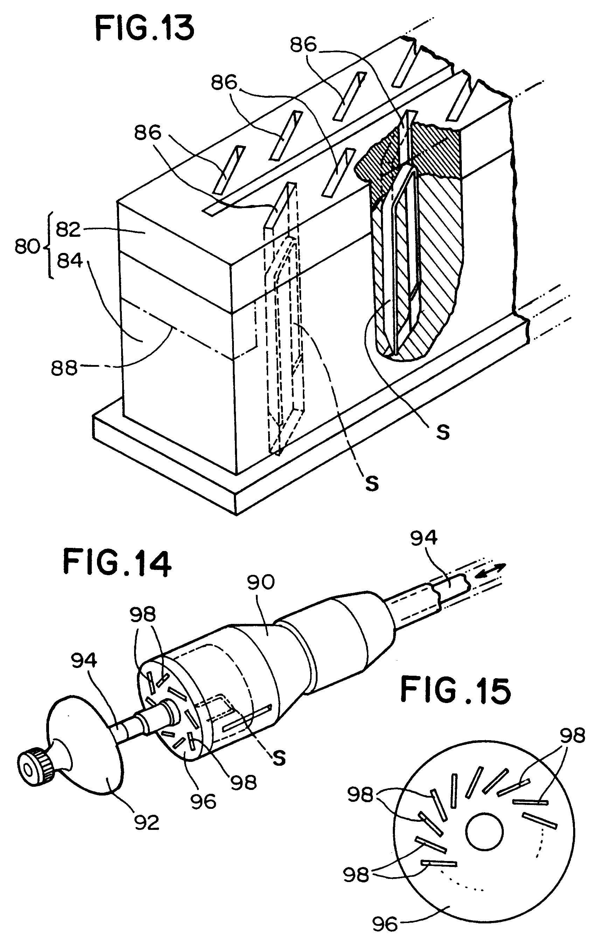

Figure 1 is a side view, partly in section, of a prior art surgical stapler; Figure 2 is a partially enlarged perspective view of Figure 1; Figure 3 is an enlarged vertical section of an anvil groove in the stapler of Figure 1; Figure 4 is a partly sectioned side view showing one embodiment of a surgical stapler according to the present invention; Figure 5 is a partially enlarged perspective view of the stapler of Figure 4; Figure 6 is a perspective view of a staple cartridge used in the stapler shown in Figure 4; Figure 7 is a sectional view of an anvil groove in another embodiment of this invention; Figures 8A and 8B are a perspective view and a sectional view taken along B-B in Figure 8A, showing the state in which bodily tissue is sutured with the staples according to this invention; Figure 9 is a partly plan view of the staple cartridge in still another embodiment of this invention; Figure 10 is a perspective view of a staple according to this invention; Figure 11 is a partially cutaway, enlarged perspective view of an anvil groove in yet another embodiment of this invention; Figure 12 is a perspective view showing a staple in a further embodiment of this invention; Figure 13 is a partially cutaway, enlarged perspective view of a staple cartridge in a still further embodiment of this invention; Figure 14 is a partly perspective view of a further embodiment of the present invention; and Figure 15 is a staple cartridge in Figure 14.

One embodiment of the surgical suturing stapler with a detachable staple cartridge according to this invention will be described hereinbelow with reference to Figures 4 and 5.

The surgical stapler comprises a first jaw 10 provided on one surface 10a thereof with a plurality of staple extruding pieces 12; a second jaw 20 having a plurality of anvil grooves 22 formed in the surface 20a opposite to the surface on which the staple extruding pieces 12 are planted on the first jaw 10; a cartridge holding member 30 having a cartridge hole 32, which is placed between the first and second jaws 10, 20; and a staple cartridge 40 having a plurality of staple chambers 42 for containing staples (S), which is detachably fitted into the cartridge hole 32 of the cartridge holding member 30.

The first and second jaws 10, 20 and cartridge holding member 30 are pivoted around an axial pin a1. The first jaw 10 is pivotally connected to a handle 14 through an axial pin a2, and the second jaw 20 is pivotally connected to a handle 24 through an axial pin a3. The handles 14, 24 are pivoted around an axial pin a4. Thus, the parts linking the pins a1-a4 constitute a lever structure shaped in a general pantograph, so that the first and second jaws 10, 20 can be forcibly closed by grasping the handles 14, 24. Though the jaws 10, 20 are respectively separated from the corresponding handles 14, 24 in this embodiment, the jaw 10 may however be integrally connected to the handle 14, and the jaw 20 may be integrally connected to the handle 24. In this structure, the aforenoted axial pins a2-a4 can be omitted.

The staple cartridge 40 which is fitted into the cartridge hole 32 in the cartridge holding member 30 is generally held by frictional force brought about by the inner surrounding surface of the cartridge hole 32. However, in order to more steadily hold the cartridge within the cartridge hole 32, a spring or other possible means for positively securing the cartridge 40 within the cartridge hole 32 may be assembled inside the cartridge holding member 30.

The cartridge holding member 30 is urged toward the first jaw 10 by a spring 26 having one end fixed on the second jaw 20 so that the space between the second jaw 20 and the cartridge holding member 30 can be made wide to facilitate to put thereinto an object such as bodily tissues to be sutured.

The staple chamber 42 in the staple cartridge 40 is substantially equal in plain geometric shape to the staple (S), so as to securely retain the staple within the staple chamber 42. The height of the staple chamber 42 is somewhat larger than that of the staple and nearly equal to that of the staple cartridge 40.

The staple chambers 42 are arranged aslant in plane relative to the lengthwise direction of the cartridge holding member as illustrated in Figure 5, and may preferably be brought closer to the adjacent ones in order to diminish the pitch at which the staples are inserted through the object to be sutured. By forming the staple chambers as close as possible, the object such as bodily surfaces can be sufficiently sutured if one of the staples loaded into the staple cartridge 40 falls off accidentally.

In this embodiment, two rows of the staple chambers 42 arranged aslant as noted above are formed across a ditch 44 for disjoining the rows of staple chambers 42.

Reference numeral 28 denotes a positioning projection formed on the leading portion of the second jaw 20. When the jaws 10, 20 are closed, the projection 28 is fitted into a hole 38 bored in the lower surface of the leading portion of the cartridge holding member 30, so that the position of the cartridge holding member 30 relative to the second jaw 20 can be decided with accuracy.

As shown in Figure 6, the staple cartridge 40 has a flange 48 which is received by a step portion 38 formed in the lower surface 30a of the cartridge holding member 30. Owing to the flange 48, the cartridge 40 is prevented from passing through the cartridge hole 32 in the cartridge holding member 30. The lower surface 40a of the staple cartridge 40 comes into parallel contact with the upper surface 20a of the second jaw 20 when the first and second jaws 10 and 20 are closed around the axial pin a1.

The anvil grooves 22 are formed in the positions which face the staple chambers 42 in the cartridge 40 fitted into the cartridge hole 32 of the cartridge holding member 30 when bringing the lower surface 40a of the cartridge 40 in face contact with the upper surface 20a of the second jaw 20. The bottom surface 22a of the anvil groove 22 formed in the second jaw 20 assumes an arc shape as shown Figure 7.

On the other hand, when the jaws 10, 20 are closed, the staple extruding pieces 12 on the first jaw 10 are plunged into the staple chambers 42 of the cartridge 40 fitted into the cartridge hole 32 of the cartridge holding member 30, consequently to discharge the staples (S) out of the staple chambers 42 toward the second jaw 20. The staples thus discharged are thrust into the anvil grooves 22 of the second jaw 20 to cause the leg portions of the staples to be bent inwardly along the bottom surfaces of the anvil grooves 22 as shown in Figure 7. If there is placed an object such as bodily tissues between the cartridge holding member 30 and the second jaw 20 when discharging the staples, the object (O) is sutured with staples as shown in Figure 8A. Since the leg portions (Sa) of the staple (S) piercing the object (O) are inwardly bent and become substantially in parallel with the plane portion (Sb) of the staple as shown in Figure 8B, so as to press the object uniformly over the entire area with the leg portions (Sa). Thus, the bodily tissue is sutured with uniform press force, to insure complete arrest of hemorrhage.

Though two rows of staple chambers 42 arranged aslant are formed in the staple cartridge 40 in the foregoing embodiment, this structure should not be understood as limitative. For example, as shown in Figure 9, the staple cartridge 50 may be provided across a disjoining ditch 54 with one row of staple chambers 52a arranged in a straight line and another row of staple chambers 52b arranged aslant. Since the tissue on the side sutured by the staples discharged from the staple chambers 52a becomes useless after operation, the staple chambers 52a may be arranged straight.

The leg portions (Sa) of the staple (S) each has a sharply pointed end as shown in Figure 10. The leg portions must be designed so as not to interfere with each other when being bent within the anvil groove 22 in the second jaw 20. To attain this purpose, there may be adopted a structure as shown in Figure 11. That is, an anvil groove 72 in a second jaw 70 is constituted by two adjoining guide grooves 72a, 72b which are staggered from each other, so that one of the legs (Sa) is guided by the guide groove 72a, and the other leg is guided by the guide groove 72b when being thrust into the anvil grooves 22. With this anvil groove, the legs of the staple are suitably bent along the respective guide grooves without colliding with each other, as indicated by the imaginary line in Figure 10.

To prevent the legs of the staple from colliding with each other when being thrust into the anvil groove formed in the second jaw, the legs of the staple may be sharpened in a wedge shape as shown in Figure 12. This staple with sharply pointed ends also facilitates to be inserted into the object such as bodily tissues to be sutured. Furthermore, it is required to make the legs (Sa) of the staple sufficiently longer than the plane portion (Sb), so that the object such as tissues can be stably and reliably sutured.

Next, a structure capable of steadily holding the staples in the staple magazine will be described with reference to Figure 13.

A staple cartridge 80 in this embodiment comprises a magnet member 82 and a cartridge body 84 of non-magnetic material. There are formed staple chambers 86 through the united magnet member 82 and cartridge body 84. The staples (S) which are generally made of metal and inserted into the staple chambers 86 are steadily held within the staple chambers 86 by magnetic attracting force produced by the magnet member 82. Thus, the staples magnetically held within the cartridge can no longer fall out of the staple chambers accidentally.

Though the staple cartridge 80 has the magnet member 82 as noted above in the illustrated embodiment, the entire staple cartridge as shown in Figure 6 may of course be made of magnetic material and magnetized.

An embodiment utilizing a cylindrical staple cartridge 96 which is applicable to suturing of tubular tissues such as an intestine or the like is illustrated in Figure 14. The cylindrical cartridge 96 has staple chambers 98 arranged aslant circumferentially and is detachably mounted onto a first jaw 90 as shown in Figure 15. A second jaw 92 is detachably secured at the leading end of a shaft 94 penetrating the assembled first jaw 90 and cartridge 96, and can open or close relative to the first jaw 90 by operating the shaft 94. Upon putting the tubular tissues to be sutured between the stapler cartridge 96 and second jaw 92, the first and second jaws 90, 92 are forcibly closed by drawing the shaft 94. While closing the first and second jaws, the staples (S) loaded within the staple cartridge 96 are discharged toward the second jaw 92 and thrust into anvil grooves formed in the second jaw 92 to cause the legs of the staples to be inserted through the tissues and bend inwardly. Thus, the tissues are steadily sutured while arresting hemorrhage as desired.

As is clear from the foregoing, the surgical stapler according to the present invention employs the detachable staple cartridge which can readily be fitted into the stapler so that the stapler can be easily and promptly loaded with suturing staples even in the middle of a surgical operation. Therefore, the stapler of this invention insures ease of handling and proves advantageous from the standpoint of safety in a surgical operation. Also, since the staple cartridge is detachable as touched upon above, the staples contained in the staple cartridge can be visually discerned with ease so as to prevent faulty suturing. Besides, by providing the staple cartridge with magnetic force, the metal staples can be securely retained within the staple cartridge so as to prevent the staples from falling out of the staple cartridge accidentally.

## Claims

1. A surgical stapler comprising a first jaw (10,90) having staple extruding pieces (12), a second jaw (20,92) having anvil grooves (22,72), and a staple magazine (30) placed between said first and second jaws, said first jaw (10,90), said second jaw (20,92) and said staple magazine being pivotally connected by an axial pin (a1,), characterized by said staple magazine being in the form of a cartridge holding member (30) having a cartridge receiving aperture (32), by a staple cartridge (40,50,80,96) having staple chambers (42,52a,86,98) into which staples (S) are insertable, said staple cartridge being detachably insertable into said cartridge receiving aperture (32) in said cartridge holding member (30), said staple chambers being located opposite to said staple extruding pieces (12) and said anvil grooves (22,72), and by said anvil grooves (22) each having an arc-shaped bottom surface (22a) and being of such a size that both leg portions of a staple (S) are bent inwardly along the bottom surface of a single respective groove (22a).

2. A surgical stapler according to Claim 1, further comprising a first handle (14) connected to said first jaw through a second axial pin (a2), and a second handle (24) connected to said second jaw through a third axial pin (a3), said first and second handles are pivoted by a fourth axial pin (a4) so as to open and close said first and second jaws (10,20) by operating said first and second handles (14,24).

3. A surgical stapler according to Claim 1 wherein said staple cartridge (40,50,80) has two rows of said staple chambers (42,86), and said staple chambers in at least one row are arranged aslant.

4. A surgical stapler according to Claim 1 wherein said staple cartridge (50) has first and second rows of said staple chambers (52a,52b), and said staple chambers (52b) in said first row are arranged aslant, and said staple chambers (52a) in said second row are arranged in a line.

5. A surgical stapler according to Claim 1 wherein said anvil groove (72) in said second jaw (20) is constituted by two adjoining guide groves (72a,72b) staggered from each other.

6. A surgical stapler according to Claim 1 wherein said staple cartridge (80) has a magnet member (82) for attracting staples (S) magnetically.

7. A surgical stapler according to Claim 1 wherein said staple cartridge (40,50) is made of magnetic material and magnetized.

8. A surgical stapler according to Claim 1 wherein said staple cartridge (96) is formed in a cylindrical shape.

9. A surgical stapler according to Claim 8 wherein said staple chambers (98) are circumferentially arranged.

10. A surgical stapler according to Claim 9 wherein said staple chambers (98) formed in said staple cartridge (96) are arranged aslant.

## Patentansprüche

1. Chirurgisches Klammernahtgerät, das einen ersten Backen (10, 90) mit Klammerausstoßteilen (12) aufweist, einen zweiten Backen (20, 92) mit Amboßnuten (22, 72) und eine Klammerspeichereinrichtung (30), die zwischen dem ersten und zweiten Backen angeordnet ist, wobei der erste Backen (10, 90), der zweite Backen (20, 92) und die Klammerspeichereinrichtung schwenkbar mittels eines axialen Stiftes (a1) verbunden sind, dadurch gekennzeichnet, daß die Klammerspeichereinrichtung die Form eines Magazinhalteteils (30) hat, welches eine Magazinaufnahmeöffnung (32) aufweist, daß ein Klammermagazin (40, 50, 80, 96) mit Klammerkammern (42, 52a, 86, 98) vorgesehen ist, in welche die Klammern (S) einlegbar sind, wobei das Klammermagazin herausnehmbar in die Magazinaufnahmeöffnung (32) im Magazinhalteteil (30) einführbar ist, wobei die Klammerkammern den Klammerausstoßteilen (12) und den Amboßnuten (22, 72) gegenüberliegend angeordnet sind, und daß die Amboßnuten (22) jeweils eine bogenförmige Bodenfläche (22a) haben und derart dimensioniert sind, daß beide Beinabschnitte einer Klammer (S) längs der Bodenfläche einer einzelnen entsprechenden Nut (22a) nach innen gebogen werden.

2. Chirurgisches Klammernahtgerät nach Anspruch 1, das ferner einen ersten Handgriff (14) aufweist, der mit dem ersten Backen über einen zweiten axialen Stift (a2) verbunden ist, und einen zweiten Handgriff (24), der mit dem zweiten Backen über einen dritten axialen Stift (a3) verbunden ist, wobei der erste und zweite Handgriff durch einen vierten axialen Stift (a4) schwenkbar gehaltert sind, so daß der erste und zweite Backen (10,20) durch Betätigen des ersten und zweiten Handgriffs (14, 24) geöffnet und geschlossen werden.

3. Chirurgisches Klammernahtgerät nach Anspruch 1, wobei das Klammermagazin (40, 50, 80) zwei Reihen von Klammerkammern (42, 86) aufweist, und die Klammerkammern in wenigstens einer Reihe schräg angeordnet sind.

4. Chirurgisches Klammernahtgerät nach Anspruch 1, wobei das Klammermagazin (50) eine erste und zweite Reihe von Klammerkammern (52a, 52b) aufweist und die Klammerkammern (52b) in der ersten Reihe schräg angeordnet sind, und wobei die Klammerkammern (52a) in der zweiten Reihe in einer Linie angeordnet sind.

5. Chirurgisches Klammernahtgerät nach Anspruch 1, wobei die Amboßnut (72) im zweiten Backen (20) von zwei benachbarten Führungsnuten (72a, 72b) gebildet sind, die zueinander versetzt angeordnet sind.

6. Chirurgisches Klammernahtgerät nach Anspruch 1, wobei das Klammermagazin (80) ein Magnetteil (82) zum magnetischen Anziehen der Klammern (S) aufweist.

7. Chirurgisches Klammernahtgerät nach Anspruch 1, wobei das Klammermagazin (40, 50) aus einem magnetischen Material besteht und magnetisiert ist.

8. Chirurgisches Klammernahtgerät nach Anspruch 1,wobei das Klammermagazin (96) zylindrisch geformt ist.

9. Chirurgisches Klammernahtgerät nach Anspruch 8, wobei die Klammerkammern (98) in Umfangsrichtung angeordnet sind.

10. Chirurgisches Klammernahtgerät nach Anspruch 9, wobei die im Klammermagazin (96) ausgebildeten Klammerkammern (98) schräg angeordnet sind.

## Revendications

1. Instrument d'agrafage chirurgical comprenant une première mâchoire (10, 90) comportant des pièces (12) d'éjection d'agrafe, une seconde mâchoire (20, 92) comportant des rainures d'enclume (22, 72), et un magasin (30) d'agrafes placé entre lesdites première et seconde mâchoires, ladite première mâchoire (10, 90), ladite seconde mâchoire (20, 92) et ledit magasin d'agrafes étant reliés de façon pivotante par une tige axiale (a1), caractérisé en ce que ledit magasin d'agrafes se présente sous la forme d'un élément (30) de support de cartouche comprenant une ouverture (32) de réception de cartouche, une cartouche (40, 50, 80, 96) d'agrafes comporte des chambres (42, 52a, 86, 98) à agrafe dans lesquelles peuvent être insérées des agrafes (S), ladite cartouche d'agrafes pouvant être insérée de façon amovible dans ladite ouverture (32) de réception de cartouche formée dans ledit élément (30) de support de cartouche, lesdites chambres à agrafe étant situées en face desdites pièces (12) d'éjection d'agrafe et desdites rainures d'enclume (22, 72), et lesdites rainures d'enclume (22) ont chacune une surface inférieure (22a) en forme d'arc et ont une taille telle que les deux parties jambes d'une agrafe (S) sont repliées vers l'intérieur le long de la surface inférieure d'une simple rainure respective (22a).

2. Instrument d'agrafage chirurgical selon la revendication 1, comprenant en outre une première poignée (14) reliée à ladite première mâchoire par une seconde tige axiale (a2), et une seconde poignée (24) reliée à ladite seconde mâchoire par une troisième tige axiale (a3), lesdites première et seconde poignées pouvant pivoter à l'aide d'une quatrième tige axiale (a4) de manière à ouvrir et à fermer lesdites première et seconde mâchoires (10, 20) par l'actionnement desdites première et seconde poignées (14, 24).

3. Instrument d'agrafage chirurgical selon la revendication 1, dans lequel ladite cartouche (40, 50, 80) d'agrafes comporte deux rangées desdites chambres (42, 86) à agrafe, et lesdites chambres à agrafe dans au moins une rangée sont disposées obliquement.

4. Instrument d'agrafage chirurgical selon la revendication 1, dans lequel ladite cartouche (50) d'agrafes comporte des première et seconde rangées de chambres (52a, 52b) à agrafe, et lesdites chambres (52b) à agrafe de ladite première rangée sont disposées obliquement, et lesdites chambres (52a) à agrafe de ladite seconde rangée sont disposées en une ligne droite.

5. Instrument d'agrafage chirurgical selon la revendication 1, dans lequel ladite rainure d'enclume (72) dans ladite seconde mâchoire (20) est constituée par deux rainures de guidage adjacentes (72a, 72b) échelonnées l'une par rapport à l'autre.

6. Instrument d'agrafage chirurgical selon la revendication 1, dans lequel ladite cartouche (80) d'agrafes comporte un élément magnétique (82) pour attirer magnétiquement les agrafes (S).

7. Instrument d'agrafage chirurgical selon la revendication 1, dans lequel ladite cartouche (40, 50) d'agrafes est faite d'un matériau magnétique et est aimantée.

8. Instrument d'agrafage chirurgical selon la revendication 1, dans lequel ladite cartouche (96) d'agrafes a une forme cylindrique.

9. Instrument d'agrafage chirurgical selon la revendication 8, dans lequel lesdites chambres (98) à agrafe sont disposées en circonférence.

10. Instrument d'agrafage chirurgical selon la revendication 9, dans lequel lesdites chambres (98) à agrafe formées dans ladite cartouche (96) d'agrafes sont disposées obliquement.
